# EUROPÄISCHE PATENTANMELDUNG

(11) **EP 1 374 929 A1**
(43) Veröffentlichungstag der Anmeldung: **02.01.2004**
(21) Anmeldenummer: 02014146.1
(22) Anmeldetag: 25.06.2002
(51) Int. Cl.: A61M 1/36

(54) **Vorrichtung zur Elimination von Gasblasen**

(71) Anmelder: Jostra AG, 72145 Hirrlingen (DE)
(72) Erfinder: van Kempen, Rutger Alexander Brest, 1826 BP Alkmaar (NL); Huntelerslag, Alexander Adriaan, 1827 RX Alkmaar (NL)
(74) Vertreter: Möbus, Daniela, Dr.-Ing.

(57) **Zusammenfassung**

Eine Vorrichtung zur Elimination von Luftblasen in extrakorporalen Flüssigkeitskreisläufen, die eine Luftblasensensoreinrichtung (10) und eine Luftblaseneliminationseinrichtung (11) aufweist und in einer Flüssigkeitsleitung (12) zwischen dem Patienten und einer Umwälzpumpe (14) für die Flüssigkeit angeordnet ist.

## Beschreibung

Die Erfindung betrifft eine Vorrichtung zur Elimination von Luftblasen in extrakorporalen Flüssigkeitskreisläufen, insbesondere in extrakorporalen Blutkreisläufen.

Insbesondere bei Operationen am schlagenden oder stillstehenden Herzen müssen die Patienten an eine Herz-Lungen-Maschine angeschlossen werden, was bedeutet, dass Blut in einem extrakorporalen Flüssigkeitskreislauf zunächst aus dem Körper heraus über die Herz-Lungen-Maschine und anschließend zurück in den Körper des Patienten gepumpt wird. Dabei muss streng darauf geachtet werden, dass keine Luft oder Blasen mit dem Blut zurück in den Körper des Patienten gelangen.

Deswegen sind bei allen extrakorporalen Kreisläufen sogenannte Blasenfallen zur Elimination von im Blut oder anderen Körperflüssigkeiten enthaltenen Luftblasen vorgesehen. Bei allen bekannten Systemen sind diese Luftblasenfallen in der Flüssigkeitszuleitung zum Körper angeordnet. Damit soll sichergestellt werden, dass unmittelbar vor Eintritt der Flüssigkeit in den Körper des Patienten keine Luftblasen mehr enthalten sind.

Das US 5,989,438 beschreibt ein aktives Blutfilter, mit dem eine Elimination von Luftblasen möglich ist.

In der Praxis hat sich jedoch gezeigt, dass Luftblasen Einrichtungen des extrakorporalen Kreislaufsystems, insbesondere Umwälzpumpen, in ihrer Funktion stören können. Gelangen zum Beispiel Luftblasen in eine Blutpumpe, so kann es bei großen Blasen sogar zu einem Aussetzen der Pumpe kommen. Kleinere Luftblasen werden durch die Pumpe in feinste Mikroblasen zerteilt, die anschließend weder detektiert noch eliminiert werden können. Diese Mikroblasen gelangen in den Blutkreislauf des Patienten und verursachen dort Embolien in den Organen.

Der vorliegenden Erfindung liegt die Aufgabe zugrunde, eine Vorrichtung zur Luftblasenelimination in extrakorporalen Kreisläufen zu schaffen, die zuverlässig alle Luftblasen aus dem Kreislaufsystem entfernt, Umwälzpumpen vor Fehlfunktionen schützt und Embolien in den Organen des Patienten verhindert.

Die Aufgabe wird mit einer Vorrichtung der eingangs genannten Art erfindungsgemäß dadurch gelöst, dass sie eine Luftblasensensoreinrichtung und eine Luftblaseneliminationseinrichtung aufweist und in einer Flüssigkeitsleitung zwischen dem Patienten und einer Umwälzpumpe für die Flüssigkeit angeordnet ist. Luftblasen werden also jetzt vor der Umwälzpumpe und vorzugsweise in der vom Patienten kommenden Flüssigkeitsleitung detektiert und eliminiert. Dadurch ist ausgeschlossen, dass Luftblasen zu Fehlfunktionen der Pumpe führen können oder durch die Pumpe derart zerkleinert werden, dass sie anschließend durch eine Luftblasenfalle nicht mehr detektiert und eliminiert werden und in den Blutkreislauf des Patienten gelangen könnten.

Zur Ausleitung eventuell vorhandener Luftblasen ist es von Vorteil, wenn die Luftblaseneliminationseinrichtung mit einem Vakuumbehälter verbunden ist, mit dessen Hilfe die Luft nach außen absaugbar ist.

Bei einer bevorzugten Ausführungsform können am Eingang und am Ausgang der Vorrichtung eine steuerbare Absperrvorrichtung für die Flüssigkeit angeordnet sein. Dadurch lässt sich die Vorrichtung gezielt ein- und ausschalten. Besonders vorteilhaft ist es dabei, wenn die Flüssigkeitsleitung eine flexible Schlauchleitung ist und die Absperrvorrichtung Schlauchklemmen sind. Handelt es sich beim extrakorporalen Kreislauf um einen Blutkreislauf, so hat diese Ausgestaltung der Absperrvorrichtungen den Vorteil, dass keine Ventilkörper oder dergleichen im Blutstrom notwendig sind. Das Blut wird also durch die Absperrvorrichtung nicht zusätzlichen Beschädigungen seiner Bestandteile ausgesetzt. Selbstverständlich können jedoch auch Ventilvorrichtungen vorgesehen werden, insbesondere dann, wenn es sich bei dem Kreislaufsystem nicht um ein Blutkreislaufsystem handelt.

Soll die Vorrichtung deaktiviert werden, ist es außerdem zweckmäßig, die Flüssigkeit durch eine Bypassleitung an der Vorrichtung vorbeizuleiten. Auch in der Bypassleitung kann eine steuerbare Absperrvorrichtung angeordnet sein, die auch hier als Schlauchklemme ausgebildet sein kann.

Die Absperrvorrichtungen können durch eine Auswerteeinrichtung der Luftblasensensoreinrichtung angesteuert werden. Werden keine Luftblasen detektiert, so wird die Flüssigkeit über die Bypassleitung an der Vorrichtung vorbeigeleitet. Anderenfalls wird die Absperrvorrichtung in der Bypassleitung geschlossen und dafür die Absperrvorrichung am Eingang der erfindungsgemäßen Vorrichtung geöffnet, sodass die Flüssigkeit die Luftblaseneliminationsvorrichtung passieren kann. Nach dem Ausleiten der Luftblasen strömt die Flüssigkeit durch die dann ebenfalls geöffnete Absperrvorrichtung am Ausgang der Luftblaseneliminationsvorrichtung wieder aus und gelangt von dort beispielsweise zu einer Umwälzpumpe und gegebenenfalls weiteren Einrichtungen wie einem Oxygenator oder dergleichen.

Die Luftblaseneliminationseinrichtung kann ein Flüssigkeitsfilter, beispielsweise ein Blutfilter, mit einer integrierten Zentrifugalpumpe sein. Die Luftblasen steigen im Filter nach oben und werden dort von der Zentrifugalpumpe von der Flüssigkeit getrennt, sodass sie nach außen abgeleitet werden können.

Die Luftblasensensoreinrichtung ist zweckmäßigerweise vor dem Abzweig der Bypassleitung in oder an der Flüssigkeitsleitung angeordnet, um den Zustand der vom Patienten her strömenden Flüssigkeit feststellen zu können.

Weitere Vorteile ergeben sich, wenn der Vakuumbehälter über eine Flüssigkeitsrücklaufleitung mit der Flüssigkeitsleitung verbunden ist, wobei die Rücklaufleitung vor der Luftblasensensoreinrichtung in die Flüssigkeitsleitung mündet. Aufgrund des Vakuums werden in dem Behälter nicht nur Luftblasen, sondern auch in kleineren Mengen Flüssigkeit mit eingesaugt. Nach Ablassen der Luftbestandteile kann die Flüssigkeit durch Aufhebung des Vakuums wieder in den Kreislauf zurückgeleitet werden. Geschieht dies vor der Sensoreinrichtung, so kann ausgeschlossen werden, dass über den Vakuumbehälter erneut Luftblasen unentdeckt in den Kreislauf gelangen können.

Die Erzeugung des Vakuums im Vakuumbehälter kann ebenfalls von der Auswerteeinrichtung der Luftblasensensoreinrichtung oder von einem Flüssigkeitsspiegelsensor in der Luftblaseneliminationseinrichtung gesteuert sein. Damit ist es möglich, das Vakuum nur dann zu erzeugen, wenn tatsächlich Luftblasen in der Flüssigkeit detektiert wurden. Nach Elimination der Luftblasen kann das Vakuum wieder aufgehoben werden.

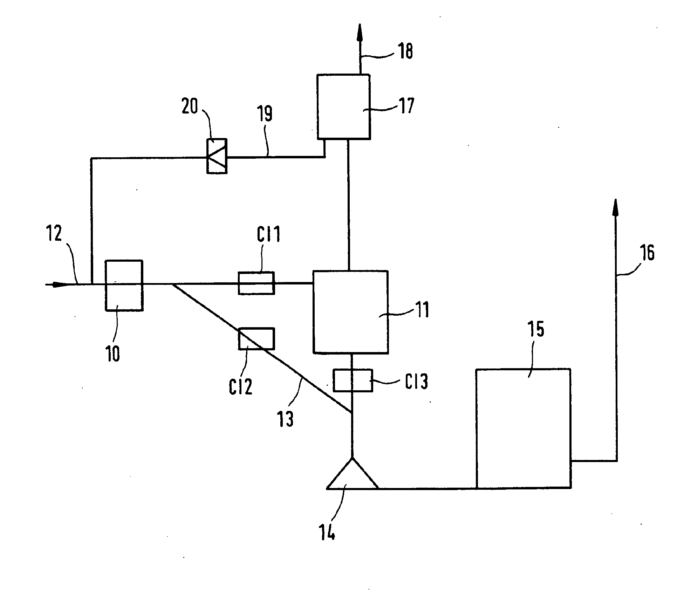

Nachfolgend wird ein bevorzugtes Ausführungsbeispiel einer erfindungsgemäßen Vorrichtung anhand der Zeichnung näher erläutert.

Die einzige Figur zeigt ein Prinzipschaltbild einer erfindungsgemäßen Vorrichtung mit einer Luftblasensensoreinrichtung 10 und einer Luftblaseneliminationseinrichtung 11, die in einer von einem Patienten kommenden Leitung 12 angeordnet sind. Am Eingang der Luftblaseneliminationseinrichtung 11 ist eine erste Absperrvorrichtung in Form einer Klemme C11 und am Ausgang eine zweite Absperrvorrichtung in Form einer Klemme C13 in der Flüssigkeitsleitung 12 angeordnet. Die Luftblaseneliminationseinrichtung 11 kann durch eine Bypassleitung 13, in der ebenfalls eine Absperrvorrichtung C12 angeordnet ist, durch die Flüssigkeit umgangen werden. Dies ist dann der Fall, wenn die Luftblasensensoreinrichtung keine Luftblasen in der Flüssigkeit detektiert. Die Flüssigkeit gelangt dann über die Bypassleitung 13 zu einer Umwälzpumpe 14 und zu einer weiteren Einrichtung 15, beispielsweise einem Oxygenator, bevor sie zu weiteren Behandlungseinrichtungen oder direkt wieder zum Körper des Patienten geleitet wird. Die Luftblasensensoreinrichtung 10 weist eine Auswerteelektronik auf, die die Absperrvorrichtungen C11, C12, C13 in Abhängigkeit vom Messergebnis ansteuert. Werden Luftblasen detektiert, so wird die Absperrvorrichtung C12 in der Bypassleitung 13 geschlossen und die Absperrvorrichtung C11 am Eingang der Luftblaseneliminationseinrichtung 11 ebenso wie die Absperrvorrichtung (13) geöffnet. Die luftfreie Flüssigkeit wird von der Umwälzpumpe 14 angesaugt. Die Luftblasen werden am oberen Ende der Luftblaseneliminationseinrichtung ausgeleitet. Die Luftblaseneliminationseinrichtung 11, die beispielsweise ein Blutfilter mit integrierter Zentrifugalpumpe oder eine Blasenfalle oder dergleichen ist, ist dazu mit einem Vakuumbehälter 17 verbunden, der die durch die Luftblaseneliminationseinrichtung 11 von der Flüssigkeit getrennten Luftbestandteile absaugt und über eine Leitung 18 nach außen leitet. Der Flüssigkeitsfluss zum Patienten ändert sich dadurch nicht. Da durch das Vakuum im Behälter 17 nicht nur Luftbestandteile, sondern auch Flüssigkeitsbestandteile mit angesaugt werden, ist der Behälter 17 über eine Leitung 19 mit einem Einwegventil 20 mit der Flüssigkeitsleitung 12 verbunden. Dabei mündet der Rücklauf 19 vor der Luftblasensensoreinrichtung 10 wieder in die Flüssigkeitsleitung 12, sodass sichergestellt ist, dass nicht über den Behälter 17 neue Luftbestandteile unentdeckt in den extrakorporalen Kreislauf gelangen.

## Patentansprüche

1. Vorrichtung zur Elimination von Luftblasen in extrakorporalen Flüssigkeitskreisläufen, insbesondere in extrakorporalen Blutkreisläufen, **dadurch gekennzeichnet, dass** sie eine Luftblasensensoreinrichtung (10) und eine Luftblaseneliminationseinrichtung (11) aufweist und in einer Flüssigkeitsleitung (12) zwischen dem Patienten und einer Umwälzpumpe (14) angeordnet ist.

2. Vorrichtung nach Anspruch 1, **dadurch gekennzeichnet, dass** die Luftblaseneliminationseinrichtung (11) mit einem Vakuumbehälter (17) verbunden ist.

3. Vorrichtung nach Anspruch 1 oder 2, **dadurch gekennzeichnet, dass** am Eingang und am Ausgang der Vorrichtung eine steuerbare Absperrvorrichtung (C11, C13) für die Flüssigkeit angeordnet ist.

4. Vorrichtung nach einem der Ansprüche 1 bis 3, **dadurch gekennzeichnet, dass** die Flüssigkeitsleitung (12) eine flexible Schlauchleitung ist und die Absperrvorrichtungen (C11, C13) Schlauchklemmen sind.

5. Vorrichtung nach einem der Ansprüche 1 bis 4, **dadurch gekennzeichnet, dass** die Flüssigkeit durch eine Bypass-Leitung (13) an der Vorrichtung vorbeileitbar ist.

6. Vorrichtung nach Anspruch 5, **dadurch gekennzeichnet, dass** in der Bypassleitung (13) eine steuerbare Absperrvorrichtung (C12) angeordnet ist.

7. Vorrichtung nach einem der Ansprüche 3 bis 6, **dadurch gekennzeichnet, dass** die Absperrvorrichtungen (C11, C12, C13) durch eine Auswerteeinrichtung der Luftblasensensoreinrichtung (10) angesteuert sind.

8. Vorrichtung nach einem der Ansprüche 1 bis 7, **dadurch gekennzeichnet, dass** die Luftblaseneliminationseinrichtung (11) ein Flüssigkeitsfilter, insbesondere ein Blutfilter, mit einer integrierten Zentrifugalpumpe ist.

9. Vorrichtung nach einem der Ansprüche 1 bis 8, **dadurch gekennzeichnet, dass** die Luftblasensensoreinrichtung (10) vor dem Abzweig der Bypassleitung (13) in oder an der Flüssigkeitsleitung (12) angeordnet ist.

10. Vorrichtung nach einem der Ansprüche 1 bis 9, **dadurch gekennzeichnet, dass** der Vakuumbehälter (17) über eine Flüssigkeitsrücklaufleitung (19) mit der Flüssigkeitsleitung (12) verbunden ist, wobei die Rücklaufleitung (19) vor der Luftblasensensoreinrichtung (10) in die Flüssigkeitsleitung (12) mündet.

11. Vorrichtung nach einem der Ansprüche 1 bis 10, **dadurch gekennzeichnet, dass** die Erzeugung des Vakuums im Vakuumbehälter (17) von der Auswerteeinrichtung der Luftblasensensoreinrichtung (10) und/oder einem Flüssigkeitsspiegelsensor der Luftblaseneliminationseinrichtung (11) gesteuert ist.

12. Verfahren zur Elimination von Luftblasen in extrakorporalen Flüssigkeitskreisläufen, insbesondere in extrakorporalen Blutkreisläufen, **dadurch gekennzeichnet, dass** die Luftblasen in einer Flüssigkeitsleitung vor einer Umwälzpumpe für die Flüssigkeit detektiert und eliminiert werden.
